Europäisches Patentamt

European Patent Office

Office européen des brevets

(11) Publication number: **0 125 073**

**A2**

# (12) EUROPEAN PATENT APPLICATION

(21) Application number: **84302818.4**

(22) Date of filing: **26.04.84**

(51) Int. Cl.³: **C 05 F 9/04**
C 05 F 11/08, C 12 N 1/20
C 12 N 1/14
//(C12N1/20, C12R1/145),
(C12N1/14, C12R1/645)

(30) Priority: **29.04.83 GB 8311811**

(43) Date of publication of application:
**14.11.84 Bulletin 84/46**

(84) Designated Contracting States:
**DE FR GB IT**

(71) Applicant: NATIONAL RESEARCH DEVELOPMENT CORPORATION
101 Newington Causeway
London SE1 6BU(GB)

(72) Inventor: Harper, Stephen Howard Travell
14 West Hill
Wantage Oxon OX12 9EF(GB)

(72) Inventor: Lynch, James Michael
12 The Drive Angmering-on-Sea
Littlehampton West Sussex BN16 1QH(GB)

(74) Representative: Percy, Richard Keith
Patent Department National Research Development
Corporation 101 Newington Causeway
London SE1 6BU(GB)

(54) Straw decomposition process.

(57) The invention relates to decomposing straw and is concerned particularly with the problem of increasing the nitrogen content of decomposed straw by inoculation of the straw with a dinitrogen-fixing anaerobe, e.g. *Clostridium butyricum*.

It has been found that the nitrogen content of the straw can be increased by co-inoculating the straw with a cellulolytic fungus which releases the cellulase enzyme extracellularly, so that it diffuses through the straw, and which satisfies certain defined requirements. A *Trichoderma harzianum* or *T. pseudokoningii* or *Sordaria alcina* is preferred as the cellulolytic fungus.

The third inoculant consumes oxygen, and forms a polysaccharide protective coat around the anaerobe, and is preferably an *Enterobacter*. A novel strain of *Enterobacter cloacae* excellent for this purpose has been deposited as NCIB 11836, and this strain and appropriate mutants and variants thereof are within the invention.

- 1 -

125206

## STRAW DECOMPOSITION PROCESS

This invention relates to a method of decomposing straw.

The practice of burning straw is attracting increasing criticism on grounds of atmospheric pollution and that the straw should be composted in order to recycle the humus and plant nutritional components to the soil. In order to make the composting of straw more attractive economically, ways are being sought of increasing the nitrogen content of the decomposed straw. Clearly, the more nitrogen that can be introduced to the soil in this way, the lower the need for inorganic fertilisers, another point important environmentally,

The nitrogen content of the straw could be increased by fixing dinitrogen from the air, by introducing a suitable nitrogen-fixing bacterium into the straw. However, the nitrogenase enzyme which takes part in $N_2$-fixation has a high ATP (energy) requirement. To supply the energy needed, in amounts which would enable the $N_2$-fixer to thrive in competition with organisms occurring naturally in the straw, it is necessary to add some other organism to the straw which is capable of breaking it down into simple carbohydrates.

Dinitrogen-fixing organisms are, of course, found in most normal soils and as long ago as 1928 P. G. Krishna, J. Amer. Soc. Agronomy 20, 511-514 (1928), showed that in the decomposition of straw other organisms supplied the energy requirements of the anaerobic $N_2$-fixers Bacillus amylobacter, which in modern nomenclature is a Clostridium butyricum, and Azotobacter chroococcum. In some experiments a fungus of the Trichoderma species was added to the straw. In others a suspension of cellulose-decomposing bacteria (not further identified) were added. It was concluded that B. amylobacter when accompanied by cellulose-decomposing bacteria fixed more dinitrogen than when accompanied by Trichoderma. B. amylobacter used the decomposition products more efficiently than Azotobacter.

U. Vartiovaara, J. Sci. Agric. Soc. Finland, 10, 241-264 (1938), studied the growth of the anaerobic $N_2$-fixers Clostridium

pasteurianum and Azotobacter, in association with cellulose-
decomposing fungi Sporotrichum carnis and Monotospora daleae.
Some $N_2$-fixation occurred when combined nitrogen was added in
order to get the associative growth started. These experiments
were carried out on media of pure technical alpha-cellulose as the
only carbon source.

Studies were carried out by H. I. Jensen, Proceedings of the
Linnean Society, NSW, Australia 66, 89-106 (1941), and H. I. Jensen
et al., ib. id., 66, 239-248 (1941) from which he concludes that
the most promising associations of microorganisms for decomposing
straw are (1) Azotobacter plus cellulose-decomposing corynebacteria
or (2) Clostridium butyricum plus any of : the aerobic cellulose-
decomposing bacterium Cellvibrio, the fungus Trichoderma koningii,
an unknown fungus "P" and actinomycetes. It was considered that a
certain degree of anaerobiosis is necessary for active $N_2$-fixation.

Clostridium species have been claimed to be the major $N_2$-fixing
members of the microorganism population of straw, by means of
acetylene reduction tests, see W. A. Rice et al., Canadian Journal
of Microbiology 18, 715-723 (1972) and C. I. Mayfield et al.,
ib. id., 20, 1503-1507 (1974).

Y. Chang and H. J. Hudson, Transactions of the British
Mycological Society 50, 649-666, (1967), describe an ecological
study of the fungi of wheat straw compost. These authors describe
fungi of some 15 different genera (none of which are germane to
the present invention) found in composts of wheat straw and grass
cuttings. The authors remark on page 663 that bacteria and
actinomycetes play an important role in composts, but give no
details of any of these organisms or their role.

More recently, P. P. Wong et al., Canadian Journal of
Microbiology, 26, 291-296 (1980) have isolated from straw a cellulolytic community including an Azospirillum species as the $N_2$-fixer.

Very little other hard information is available about the
microorganisms present in straw or their effects. J. M. Lynch
et al., Journal of General Microbiology 127, 231-236 (1981)
isolated microorganisms from soil and tested their individual
ability to decompose straw cellulose. There were 10 fungi, 4 yeasts and 3 bacteria. The community of fungi, <u>Cladosporium</u> <u>cladosporoides</u>, 2 <u>Fusarium</u> spp., <u>Geotrichum</u> <u>candidum</u>, <u>Gliocladum</u> <u>roseum</u>, <u>Mucor</u> <u>hiemalis</u>, <u>Penicillium</u> <u>chrysogenum</u>, <u>Pencillium</u> <u>cyclopium</u>, <u>Trichoderma</u> <u>hamatum</u> and <u>Trichoderma</u> <u>viride</u>, grew on straw. Only one or two of the yeasts grew and none of the bacteria, which were <u>Bacillus</u> <u>cereus</u>, <u>Hafnia</u> <u>alvei</u> and <u>Pseudomonas</u> <u>maltophilia</u>. An assay for cellulose-producing activity showed great variations according to the kind of cellulose substrate used and was inconclusive.

A paper by J. M. Lynch and L. F. Elliott appeared in Soil Biology and Biochemistry <u>15</u>, 221-222 (1983), issued 25th April 1983, concerned with minimising the phytotoxicity of decomposed wheat straw. In experiments, finely ground wheat straw was inoculated with various microorganisms isolated from straw viz. (a) <u>Azotobacter</u>, (b) <u>Enterobacter</u> <u>cloacae</u>, (c) <u>Mucor</u> <u>plumbeus</u>, (d) <u>Penicillium</u> <u>purpurescens</u>, (e) <u>Trichoderma</u> <u>harzianum</u>, and the combinations (a) + (e), (c) + (d) and (b) + (e), and cultured in a liquid medium. The effect of microbial decomposition of wheat straw after 1 month on its phytotoxicity to wheat seedlings was expressed in terms of the relative amounts of root extension of the seedlings. The results for straw which had been inoculated were not significantly different from control straw which was uninoculated and there were no significant differences in the results between different inocula. It was concluded, in particular, that adding cellulolytic fungi to straw seems to offer no particular advantage. The paper by J. M. Lynch and L. F. Elliott says that the microorganisms "had been isolated as dominant colonists of straw", but Dr. Lynch has since explained that it was not intended to imply that every organism listed is dominant in straw, merely that collectively they are elements of a dominant community.

A companion paper by the above authors appeared in Applied and Environmental Microbiology, <u>45</u>, 1398-1401 (1983) issued 8th April 1983. It concerns stabilisation of the soils of the Pacific Northwest of the USA, which are partly volcanic in origin and were covered with volcanic ash following the volcanic eruption of Mount St. Helens in 1980. Tests were made to see whether addition of straw to the unstable soil would improve soil stability. Experiments were carried out by inoculating finely ground wheat straw with the same microorganisms (a) to (e) and combinations as above, and culturing the straw in a liquid medium. The addition of straw degraded for 19 or 25 days to volcanic ash and soils from the region increased the stability of the soil to water erosion, but there was no consistent advantage obtained by inoculating the straw. The uninoculated control sample again performed just as well.

In another recent paper, J. M. Lynch and S. H. T. Harper, Journal of General Microbiology 129, 251-253 (1983), inoculated wheat straw with the dinitrogen-fixing anaerobe Clostridium butyricum and with the cellulolytic fungus Penicillium corylophilum and obtained a respectable gain in the nitrogen content of the straw.

It has now been found that decomposition of straw is accelerated by providing it with three microoganisms. The first is bacterial, a dinitrogen-fixing anaerobe such as C. butyricum or another dinitrogen-fixer of comparable or greater efficiency. The second is a cellulolytic fungus which is (a) capable of releasing cellulase extracellularly, so that it diffuses through straw, (b) capable of growing well on straw, and (c) not pathogenic to crops, preferably Trichoderma harzianum or hamatum. The third microorganism is a polysaccharide-producer. Its main role is to consume oxygen from the immediate environment of the anaerobe and surround the anaerobe with a coat of polysaccharide, both of which functions help to maintain anaerobic the micro-environment of the anaerobe. Preferably it also provides a growth factor required by the C. butyricum. (For this purpose removal of an inhibitory factor and provision of a growth factor are regarded as synonymous.) One particularly useful class of third microorganism in the invention is an Enterobacter cloacae which has been isolated from natural sources and deposited as a patent culture, under the provisions of the Budapest Treaty, at the National Collection of Industrial Bacteria, Torry Research Station, P.O. Box 31, 135 Abbey Road, Aberdeen AB9 8DG on 3rd March, 1983 as NCIB 11836.

The invention includes a method of decomposing straw which comprises inoculating it with

(1) a dinitrogen-fixing anaerobic bacterium capable of growing on cellobiose and producing nitrogenase with at least the efficiency of a Clostridium butyricum (measured in terms of nitrogenase activity produced per unit weight of straw);

(2) a cellulolytic fungus which satisifies the cellulose agar clearing test and growth on straw tests hereinafter defined and which is not pathogenic to crops and

(3) a polysaccharide-producing microorganism which consumes oxygen from the immediate enviroment of the anaerobic bacterium and surrounds it with a coat of polysaccharide.

A kit of these microorganisms for use in the method is included in the invention. In such a kit, each microorganism is kept in a separate container. Additionally, the novel polysaccharide-producing Enterobacter cloacae NCIB 11836 and mutants and variants thereof (appropriate to the objectives herein described) are per se part of the invention and the invention includes particularly a biologically pure culture thereof.

Although straw which is being composted becomes aerated, it is capable of supporting the growth of dinitrogen-fixing anaerobes such as Clostridium butyricum. The dinitrogen-fixing anaerobe should be capable of growing on the cellobiose produced from straw. Clostridium butyricum meets this requirement but other Clostridia do not.

The preferred anaerobe is Clostridium butyricum, which is an obligate anaerobe which needs a certain low redox potential or $O_2$-free environment in which to grow, as well as certain growth factors. The third inoculant helps to maintain the desired environment and contributes to the supply of growth factor. Clostridium butyricum NCIB 7423 is on ordinary scientific deposit at the National Collection of Industrial Bacteria, Aberdeen, Scotland and is eminently suitable, although other strains of Clostridium butyricum can be isolated from straw and used.

Most straw contains sufficient native Clostridium butyricum, but since one cannot be certain that it will be present in any particular instance, it is a sensible precaution to inoculate the straw with $\underline{C}$. $\underline{butyricum}$.

The second component is a cellulolytic fungus and preferably conforms to the following definition:

(a) $\underline{Cellulose\ clearing\ test}$

When a 3 mm diameter disc cut from the edge of fungal colonies growing on malt agar is placed on cellulose agar of the following composition:

| Ingredient | Grams/litre |
|---|---|
| Cellulose | 10 |
| $KH_2PO_4$ | 1.0 |
| KCl | 0.5 |
| $MgSO_4.7H_2O$ | 0.2 |
| $CaCl_2$ | 0.1 |
| $(NH_4)_2SO_4$ | 0.5 |
| Thiamine hydrochloride | 0.001 |
| Agar | 20 |

for a period of 4 weeks at $20^{o}C$, the fungus clears the cellulose agar to a depth of 4 mm or more measured from the upper surface of the cellulose agar.

(b) $\underline{Growth\ on\ straw\ test}$

When wheat straw ground to lengths of 0.5 to 2.0 mm (1 g) is added to Dalton and Postgate nitrogen-free mineral salt medium (100 ml) amended with 1 g/litre ammonium chloride, the straw is heat-sterilised at $121^{o}C$ and 103 kPa for 15 minutes and inoculated with a disc 5 mm in diameter cut from the edge of fungal colonies growing on malt agar, the inoculated straw is incubated for 4 weeks on an orbital shaker at 180 rev/minute at $20^{o}C$, the straw is removed on filter paper, rinsed with distilled water and dried at $60^{o}C$ for 16 hours, the weight loss of the straw is greater than that obtained from $\underline{Penicillium}$ $\underline{corymbiferum}$ IMI 284723, deposited at the Commonwealth Mycological Institute, Ferry Lane, Kew, Surrey TW9 3AF as an ordinary scientific deposit, in the same test.

(c) Pathogenicity

The fungus is not pathogenic to crops (and therefore is not any of the commonly encountered <u>Fusarium</u> species), particularly not cereal crops.

The Dalton and Postgate medium referred to herein is according to the paper by H. Dalton and J. R. Postgate, Journal of General Microbiology, <u>54</u>, 463-473 (1969), and has the following composition:

| Ingredient | Grams/litre |
|---|---|
| $K_2HPO_4$ | 0.64 |
| $KH_2PO_4$ | 0.16 |
| $NaCl$ | 0.2 |
| $MgSO_4.7H_2O$ | 0.2 |
| $CaCl_2$ | 0.1 |
| $FeSO_4.7H_2O$ | 0.0025 |
| $H_3BO_3$ | 0.0029 |
| $CoSO_4.7H_2O$ | 0.0012 |
| $CuSO_4.5H_2O$ | 0.0001 |
| $MnCl_2.4H_2O$ | 0.00009 |
| $Na_2MoO_4.2H_2O$ | 0.0025 |
| $ZnSO_4.7H_2O$ | 0.0012 |
| Nitrilotriacetic acid | 0.1 |

The preferred fungal components are <u>Trichoderma</u> <u>harzianum</u>, <u>Trichoderma</u> <u>hamatum</u> and <u>Sordaria</u> <u>alcina</u>. Others satisfying the definition are <u>Acremonium</u> <u>persicinum</u>, <u>Penicillium</u> <u>janthinellum</u>, <u>Trichoderma</u> <u>viride</u> and an un-named fungus herein designated "C3/5".

<u>Trichoderma</u> <u>reesei</u>, a mutant of <u>T.</u> <u>viride</u>, is unsatisfactory for use in the invention.

The following Table 1 reports the results of the above tests (a) and (b) for 19 fungi found in straw. <u>Fusarium</u> species (<u>F.</u> <u>avenaceum</u>, <u>culmorum</u>, <u>equiseti</u>, <u>solani</u> and <u>oxysporum</u>) passed one or both tests but, being pathogens, are not of practical use. It will be seen that most <u>Penicillium</u> species perform poorly and <u>P.</u> <u>corylophilum</u>, at one time considered promising, is not within the invention. Those fungi passing both tests, as indicated by asterisks in each column, are useful in the invention. For better identification of the preferred strains and some comparative fungi, scientific deposits have been made at the Commonwealth Mycological Institute and IMI numbers are shown in the Table. All these deposits are available to the public. Other strains of the same species are usable. Many are catalogued by the CMI.

TABLE 1

Performance of fungal isolates in tests

(* = satisfies test;  only those giving asterisks in both columns are within the invention)

| | Wt. loss from straw (mg/g) | Clearing of celluose agar (mm) |
|---|---|---|
| Acremonium persicinum (IMI 284720) | 305* | 4* |
| Botryotrichum piluliferum (IMI 284721) | 145* | 0 |
| Chaetomium globosum | 240* | 0 |
| Geotrichum candidum | 50 | 0 |
| Penicillium chrysogenum | 99 | 6* |
| Penicillium corylophilum (IMI 284722) | 69 | 0 |
| Penicillium cyclopium | 100 | 0 |
| Penicillium echinulatum | 120 | 0 |
| Penicillium corymbiferum (IMI 284723) | 125 | 5* |
| Penicillium janthinellum (IMI 284724) | 240* | 6* |
| Penicillium purpurascens | 61 | 8* |
| Penicillium simplicissimum | 110 | 6* |
| Penicillium spinulosum | 95 | 0 |
| Penicillium viridicatum | 115 | 0 |
| Sordaria alcina (IMI 267236) | 267* | 5* |
| C 3/5 (IMI 284729) | 351* | 6* |
| Trichoderma hamatum (IMI 284726) | 250* | 11* |
| Trichoderma harzianum (IMI 275950) | 238* | 10* |
| Trichoderma viride (IMI 284728) | 236* | 15* |

In assessing the performance of the failures it should be borne in mind that many only cleared the agar at the very end of the 4 week period. Therefore although some of the failures pass or only just fail the clearing test and fail only on weight loss from straw, their clearing test results are mainly not really as satisfactory or near-satisfactory as might appear.

Preferably the cellulose clearing is to a depth of 5 mm or more. Preferably the growth on straw test gives a weight loss at least equal to that of Botryotrichum piluliferum IMI 284721, but most preferably at least equal to that of Trichoderma harzianum, hamatum, or viride.

Trichoderma harzianum is particularly advantageous in that it competes well with a wide range of pathogens such as Fusarium spp. which are common on straw, it grows rapidly so that only very small amounts of inoculum are needed to give a good spread of fungus throughout the straw, and it spores readily, making inoculum production easy.

The third micoorganism is preferably of the genus Enterobacter, especially E. cloacae, and most especially the E. cloacae NCIB 11836 mentioned above. Alternatives are Azotobacter spp. and those yeasts which are strong polysaccharide producers. The yeasts can be those found naturally or genetically manipulated and it is contemplated that by appropriate genetic manipulation their polysaccharide-producing and/or oxygen-consuming properties will be enhanced.

Any amounts and relative proportions of the three microorganisms effective to secure their respective functions as stated above are usuable. Suggested concentrations of the bacterial inoculants are from $10^3$ to $10^7$ colony-forming units per gram of straw. The preferred range is from $10^4$ to $10^6$. It would not be desirable, in general, to use more organisms than $10^6$ c.f.u./g, since this would damage the main objective of the process in relying on growth of bacteria to bring about the nitrogen gain. The $10^6$ level typically represents about 1% or less of the final bacterial population.

The fungus spreads outwardly from a point to cover the straw. It needs merely to cover it. It is not particularly desirable to provide large amounts of fungus relative to the numbers of bacteria, since it would then undesirably compete with them. The requirement is merely that the fungus must completely colonise the straw. The more active fungi, such as the Trichodermas and Sordaria have very rapid growth rates, so that ten spores or possibly less will usually be sufficient. On the other hand, the _Penicillium_ species do not extend so well across the straw and a higher amount such as 100 spores is a more desirable minimum. In general, the usable range is from 10 to $10^5$ spores per gram of straw. The preferred range is $10^2$ to $10^4$. It might be possible to use the fungus in the form of mycelium, in which case an equivalent concentration in terms of mycelial mass is suggested, which for the _Trichodermas_ is typically in the range of $1.4 \times 10^{-7}$ to $1.4 \times 10^{-3}$/mg (typically 140 nanograms of mass = 1 spore equivalent). Similar calculations can be made for the other fungi.

The inocula are preferably stored in separate containers. The anaerobic dinitrogen-fixer and cellulolytic fungus can be provided as spores, to be reconstituted, e.g. in powder form. The _Enterobacter_ could be provided on a peat-based or compost-based medium. Alternatively the inocula can be supplied as sprayable liquids, obtainable from liquid fermentations. The kit can also include other organisms of possible benefit and a supply of any trace elements of which a deficiency is suspected. For example, a _Trichoderma_ cellulolytic fungus might have a requirement for cobalt or manganese and a _C. butyricum_ dinitrogen-fixing anaerobe a requirement for iron and molybdenum. Thus, a mixture of mineral salts will usually be provided. If desired these components of the kit can be mixed before use and formulated as a ready-to-use inoculum of limited storage life, on a substrate of chopped straw or green manure or as a sprayable liquid.

One method of application is to heap the straw in a corner of the field or in windrows and then treat it. Care should be taken to avoid excessive heating, by modifying stack design, and to avoid drying (e.g. by covering with plastic sheeting). Although this approach lends itself to good control, its cost may be excessive for arable useage. This approach might be preferred if the compost so produced were to be sold off-farm as a horticultural compost or used on another field.

An alternative method is to chop the straw immediately after harvest, spray with inoculum and incorporate the straw in the soil surface. This would conserve moisture around the straw. A major

0125073

- 11 -

advantage is that it would add very little cost to farm operations, since the treatment sprayer could be attached to the straw chopper.

Another possible application is to treat the straw in bales wrapped in plastic sheeting, and use the carbon dioxide liberated by the straw. (It can be piped into glasshouses for growing cucumbers, which like a high carbon dioxide environment.) Since the decomposed straw can afterwards be bagged and sold as compost, this application gives two products of added value from the straw decomposition.

The pH of the straw to be decomposed is preferably from 5.5 to 6.5. The cellulase-producing activity generally works best at lower pH. At lower pH than about 5.5, especially lower than 5, the bacteria will not grow well. At a pH much above 6.5 there is a danger that one fungus present in the straw will dominate the added cellulolytic fungus. For example, Coprinus cinereus present in straw would dominate a Trichoderma.

The straw may be from any of the major cereal crops, e.g. wheat, barley, maize, oat, or rice, or from oilseed rape. It should be kept moist, e.g. have a water content equal to 50% to 100% by weight of its maximum water-holding capacity (as determined by soaking the straw in water for 24 hours).

The temperature of the straw will normally be from 15 to 30$^{\circ}$C. Any temperature tolerable to the microorganisms will in general suffice.

It might be desirable to add inorganic nitrogen as a "primer" to aid straw decomposition, particularly in the form of an ammonium salt such as the chloride or sulphate.

Trichoderma spp. have been proposed as biocontrol agents to suppress the growth of pathogenic fungi. T. hamatum, T. harzianum and T. viride are examples. It is a valuable additional feature of the invention to employ the dinitrogen-fixing bacterium and the polysaccharide producer in the culture of a Trichoderma species of the kind useful as a biocontrol agent. The extraordinary degree of co-operation between three microorganisms lends itself to such use. Accordingly, the invention further provides an artificial mixture of microorganisms comprising (1) a dinitrogen-fixing anaerobic bacterium as defined above, (2) a _Trichoderma_ bicontrol agent and (3) a polysaccharide-producing microorganism as defined above. It also includes a method of cultivation of a _Trichoderma_ bicontrol agent characterised by incorporating in the cultivation medium the other microorganisms (1) and (3). As before, any proportions effective to secure co-operation between the microorganisms to fulfil their stated function can be used.

The following Examples illustrate the invention.

## EXAMPLE 1

### Preparation of inocula

_Clostridium_ _butyricum_ NCIB 7423 was cultured on agar of composition: sucrose (10 g/litre), agar (15 g/litre) and Dalton and Postgate medium in an anaerobic cabinet containing carbon dioxide, hydrogen and nitrogen (10:10:80, by volume) at $25^{\circ}C$. Cells and spores were harvested after 14 days into sucrose-free medium amended with cysteine (0.5 g/litre) and counted under the microscope.

The _Enterobacter_ _cloacae_ NCIB 11836 was cultured in shake flasks on nutrient broth for 7 days at $20^{\circ}C$. The cells were spun down, washed and re-suspended in a sucrose-free medium, i.e. the Dalton and Postgate medium above, without sucrose. Cell numbers were again determined directly.

The _Trichoderma_ _harzianum_ IMI 275950 was grown on 'split' plates made by causing molten malt agar (5 ml), to cool as a slope at one side of the plate and molten water agar (5 ml) to cool with the plate horizontal. Isolates were inoculated into the malt agar and then left to grow across the water agar. Discs (3 mm) were cut from the water agar for use as straw inocula. This method provided a simple technique to prepare fungal inocula free from contamination by components of the malt agar.

### Preparation of straw bottles

Wheat straw was hammer-milled and sieved (0.5 to 2 mm) and sub-samples (1 g) weighed into Universal bottles. Nitrogen-free mineral salt solution (10 ml), see H. Dalton and J. R. Postgate, supra, was added and the bottles sterilised by autoclaving. Bacteria were added as a suspension, in an amount of $10^7$ cells and/or spores per gram of straw, and mixed into the straw. The Trichoderma harzianum fungi were inoculated as discs of agar into the straw surface. The bottles were then incubated at $20^{\circ}C$ for 4 weeks.

### Tests carried out

The straw bottles were inoculated with various organisms and combinations of organisms, as detailed in the Table below. The straw was analysed for gain in dinitrogen after a 4 week incubation period. Dinitrogen analysis was by a combustion method, in which the liberated oxides of nitrogen were reduced to dinitrogen gas, which was measured. Measurements were made on the straw bottles inoculated with the various organisms, as well as on replicate bottles which were uninoculated. Dinitrogen gains are shown in the Table 2 below

### TABLE 2

| Organism(s) | Nitrogen gain (mg/g straw) |
| --- | --- |
| Enterobacter cloacae | 3.9 |
| Clostridium butyricum | 3.7 |
| Trichoderma harzianum + Clostridium butyricum | 5.1 |
| Trichoderma harzianum + Clostridium butyricum + Enterobacter cloacae | 5.4 |
| Trichoderma harzianum + Enterobacter cloacae | 3.9 |

The bacteria alone fixed dinitrogen into straw, since the water-soluble components of straw provide available substrates. The cellulolytic activity of Trichoderma harzianum makes further substrate material available from the cellulose and hemicellulose components of the straw, but only the Clostridium butyricum appears able to use these in dinitrogen fixation.

The best result was obtained using the _Enterobacter cloacae_ in combination with the _Trichoderma harzianum_ and _Clostridium butyricum_ and the difference between this result and that obtained using the _Trichoderma harzianum_ and _Clostridium butyricum_ combination alone is a significant one. While the increase in dinitrogen gain in the presence of _Enterobacter cloacae_ might seem small, it should be emphasised that in this test cellulolysis is limited in comparison with composts and therefore the microbial growth and demand for growth factors is also limited. The amount of cellulose decomposed is less than half of that which is decomposed in composts.

EXAMPLE 2

This Example describes a trial of another combination of microorganisms in decomposing straw, using _S. alcina_ as the fungal component disposing of the straw in soil, and growing wheat seedlings.

Straw was composted using the column incubation method described previously by J. M. Lynch and S. H. T. Harper, J. Gen. Microbiology, _129_, 251-253, (1983), but with different inocula. Both composts were produced from wheat (var. Avalon) straw of the 1982 UK harvest inoculated with about $10^6$ spores and cells per gram of straw of _Clostridium butyricum_, _Sordaria alcina_, and _Enterobacter cloacae_ NCIB 11836.

Composts or straw were mixed with a silt loam soil (Hamble Series) and placed in pots each containing 8 g straw (or the composted residue from that quantity) and 800 g (dry weight) of soil. Wheat seed (10 per pot var. Maris Huntsman, 1980 harvest) were sown and thinned to the 5 largest seedlings after 7 days. Plants were grown at $18^{\circ}C$ in a growth cabinet with a 16 hour day/8 hour night regime. Plants were harvested after 24 days.

In duplicate experiments 336 mg $NH_4Cl$/pot was added at the time of sowing the seedlings. (This amount brings the straw C:N ratio to 30:1 which would compensate for soil nitrogen immobilised into straw.)

- 15 -

Table 3 below shows the results.

TABLE 3

| | Shoot dry weights (mg/plant) | |
| --- | --- | --- |
| | Without added N | N added at sowing* |
| Soil only | 323 | 518 |
| Soil + undecomposed 1982 straw | 128 | 378 |
| Soil + compost I (1982 straw) | 306 | 490 |

Least significant difference (P = 0.05) = 31

Similar experiments using composted 1983 wheat straw gave poorer results, but it is considered that the 1983 straw is atypical, perhaps because of the unusual weather conditions.

EXAMPLE 3

This Example illustrates other combinations of microorganisms using a variety of different fungal components.

Wheat straw (20 g var. Avalon 1983 harvest) was packed into 500 ml medical flat bottles and moistened with Dalton and Postgate nitrogen-free mineral salts solution (20 ml). After 4 hours further Dalton and Postgate solution (20 ml) was added. The latter contained the microbial inoculum and inorganic nitrogen (0.5 g $(NH_4)_2SO_4$/litre).

Inocula of Clostridium butyricum were grown in static liquid medium containing sucrose (10 g/l) and Dalton and Postgate solution. Bottles of the medium were inoculated from plate cultures and incubated for 24 hours at 25°C in an anaerobic cabinet. A cell suspension of $10^7$/ml was prepared by dilution with mineral salts solution amended with cysteine (0.5 g/l).

Enterobacter cloacae was cultured on malt agar (2 days, 20°C) and cell suspensions prepared by the same method as for C. butyricum but omitting cysteine from the medium.

Fungi were cultured on malt agar (7 days, 20°C) and spores harvested by sterile inoculation loop into Dalton and Postgate solution. A spore suspension with the same optical density as the bacterial suspensions was prepared.

Inocula were added to give $10^6$ bacteria/g straw and an equivalent dry weight of fungal spores as judged by optical density.

Results are shown in Table 4 below.

TABLE 4

|  | N gain mg/g | % weight loss |
|---|---|---|
| Uninoculated | 1.24 | 8.1 |
| C. butyricum | 1.04 | 8.6 |
| Enterobacter cloacae | 1.27 | 8.5 |
| C. butyricum + E. cloacae | 1.30 | 12.4 |
| Trichoderma hamatum | 1.24 | 18.9 |
| T. hamatum + C. butyricum | 1.41 | 18.4 |
| T. hamatum + E. cloacae | 2.05 | 19.7 |
| T. hamatum + C. butyricum + E. cloacae | 1.98 | 18.9 |
| Trichoderma harzianum | 1.34 | 16.6 |
| T. harzianum + C. butyricum | 1.49 | 17.8 |
| T. harzianum + E. cloacae | 2.15 | 18.2 |
| T. harzianum + C. butyricum + E. cloacae | 2.02 | 17.3 |
| Sordaria alcina | 1.13 | 18.5 |
| S. alcina + C. butyricum | 1.58 | 19.5 |
| S. alcina + E. cloacae | 1.56 | 16.8 |
| S. alcina + C. butyricum + E. cloacae | 1.94 | 16.8 |
| Penicillium corylophilum | 1.40 | 9.9 |
| P. corylophilum + C. butyricum | 1.35 | 12.1 |
| P. corylophilum + E. cloacae | 1.28 | 10.0 |
| P. corylophilum + C. butyricum + E. cloacae | 1.53 | 11.7 |
| Least significant difference (P = 0.05) | 0.43 | 3.3 |

CS35

CLAIMS

1.  A method of decomposing straw which comprises inoculating it with

(1)   a dinitrogen-fixing anaerobic bacterium capable of growing on cellobiose and producing nitrogenase with at least the efficiency of a <u>Clostridium butyricum</u> (measured in terms of nitrogenase activity produced per unit weight of straw);

(2)   a cellulolytic, cellulase-producing fungus capable of releasing cellulase extracellularly so that it diffuses through straw and further defined as follows:

(a)   <u>Cellulose clearing test</u>

when a 3 mm diameter disc cut from the edges of fungal colonies growing on malt agar is placed on cellulose agar of the following composition:

| Ingredient | Grams/litre |
|---|---|
| Cellulose | 10 |
| $KH_2PO_4$ | 1.0 |
| KCl | 0.5 |
| $MgSO_4.7H_2O$ | 0.2 |
| $CaCl_2$ | 0.1 |
| $(NH_4)_2SO_4$ | 0.5 |
| Thiamine hydrochloride | 0.001 |
| Agar | 20 |

for a period of 4 weeks at $20^oC$, the fungus clears the cellulose agar to a depth of 4 mm or more measured from the upper surface of the cellulose agar;

(b)   <u>Growth on straw test</u>

when wheat straw ground to lengths of 0.5 to 2.0 mm (1 g) is added to Dalton and Postgate nitrogen-free mineral salt medium (100 ml) amended with 1 g/litre ammonium chloride, the straw is heat-sterilised at $121^oC$ and 103 kPa for 15 minutes and inoculated with a disc 5 mm in diameter cut from the edge of fungal colonies growing on malt agar, the inoculated straw is incubated for 4 weeks on an orbital shaker at 180 rev/minute at $20^oC$, the straw is removed on filter paper, rinsed with distilled water and dried at $60^oC$ for 16 hours, the weight loss of the straw is greater than

0125073

- 18 -

that obtained from <u>Penicillium corymbiferum</u> IMI 284723, in the same test and

    (c)  <u>Pathogenicity</u>

    the fungus is not pathogenic to crops;  and

    (3)  a polysaccharide-producing microorganism which consumes oxygen from the immediate environment of the anaerobic bacterium and surrounds it with a coat of polysaccharide.

2.   A method according to Claim 1 wherein the nitrogen-fixing anaerobic bacterium is a <u>Clostridium butyricum</u>.

3.   A method according to Claim 1 or 2 wherein the cellulolytic fungus is a <u>Trichoderma harzianum</u>, <u>Trichoderma hamatum</u> or <u>Sordaria alcina</u>.

4.   A method according to Claim 1 or 2 wherein the cellulolytic fungus is an <u>Acremonium persicinum</u>, <u>Penicillium janthinellum</u>, <u>Trichoderma viride</u> or is C 3/5 IMI 284729.

5.   A method according to any preceding claim wherein the polysaccharide-producing microorganism is an <u>Enterobacter</u>.

6.   A method according to Claim 5 wherein the <u>Enterobacter</u> is <u>Enterobacter cloacae</u> NCIB 11836 or a mutant or variant thereof.

7.   A method according to any preceding claim wherein the method is carried out at a temperature of from 15 to $30^{o}C$, pH of from 5.5 to 6.5 and using straw which is wet to at least 50% of its maximum holding capacity of water.

8.   A method according to any preceding claim wherein the concentrations of inoculants added are from $10^3$ to $10^7$ c.f.u. or equivalent of the nitrogen-fixing bacterium (1) and of the polysaccharide-producing microorganism (3) and from $10^2$ to $10^5$ spores or equivalent of the cellulolytic fungus (2), all per gram of straw.

9.   A kit of microorganisms useful in the method claimed in Claim 1, which kit comprises

    (1)  a nitrogen-fixing anaerobic bacterium as defined in Claim 1 or 2;

    (2)  a cellulolytic fungus as defined in Claim 1, 3 or 4; and

(3)  a polysaccharide-producing microorganism which consumes oxygen as defined in Claim 1, 5 or 6.

10.  <u>Enterobacter</u> <u>cloacae</u> NCIB 11836 and mutants and variants thereof.

11.  An artificial mixture of microorganisms comprising

(1)  a nitrogen-fixing anaerobic bacterium as defined in Claim 1 or 2;

(2)  a biocontrol agent of the genus <u>Trichoderma</u>;  and

(3)  a polysaccharide-producing microorganism which consumes oxygen, as defined in Claim 1, 5 or 6.

12.  A method of cultivation of a biocontrol agent of the genus <u>Trichoderma</u> characterised by incorporating in the cultivation medium

(1)  a nitrogen-fixing anaerobic bacterium as defined in Claim 1 or 2;  and

(2)  a polysaccharide-producing microorganism which consumes oxygen, as defined in Claim 1, 5 or 6.